Europäisches Patentamt
European Patent Office
Office européen des brevets

Numéro de publication: **0 384 811 A1**

# DEMANDE DE BREVET EUROPEEN

Numéro de dépôt: 90400428.0

Date de dépôt: 16.02.90

Int. Cl.5: C07D 333/24, C07F 9/40, G02F 1/35

Priorité: 22.02.89 FR 8902271

Date de publication de la demande: 29.08.90 Bulletin 90/35

Etats contractants désignés: AT BE CH DE DK ES FR GB GR IT LI LU NL SE

Demandeur: RHONE-POULENC CHIMIE
25, quai Paul Doumer
F-92408 Courbevoie Cédex(FR)

Inventeur: Leising, Frédéric
11, allée des fauvettes, La Condamine
F-69440 Mornant(FR)
Inventeur: Meyrueix, Rémi
112, Cours Albert Thomas
F-69008 Lyon(FR)
Inventeur: Mignani, Gérard
2, avenue des Frères lumière
F-69008 Lyon(FR)

Mandataire: Ricalens, François et al
RHONE-POULENC CHIMIE Service Brevets
Chimie 25, quai Paul Doumer
F-92408 Coubevoie(FR)

## Composés thiophéniques actifs en optique non linéaire, matériaux et dispositifs les contenant.

La présente invention concerne de nouveaux composés thiophéniques de formule générale :

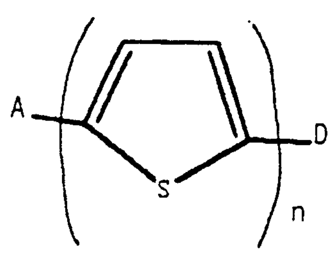

dans laquelle :
n est un nombre entier compris entre 1 et 4
A est un groupement accepteur d'électrons
D est un groupement donneur d'électrons.

Ces composés présentent une forte hyperpolarisabilité et une excellente stabilité photochimique.

Ces composés sont notamment utiles dans les dispositifs optiques ou électrooptiques pour leur activité en optique non linéaire.

EP 0 384 811 A1

## COMPOSES THIOPHENIQUES ACTIFS EN OPTIQUE NON LINEAIRE, MATERIAUX ET DISPOSITIFS LES CONTENANT

L'invention concerne des nouveaux composés thiophéniques présentant notamment une activité en optique non linéaire.

Elle se rapporte plus particulièrement à des composés thiophéniques hyperpolarisables pouvant, par exemple, notamment être ajoutés dans une matrice pour former un constituant d'un dispositif électrooptique ou optique.

Comme l'indiquent J. Zyss et I. Ledoux dans l'article paru dans "L'Echo des Recherches", 1er trimestre 1987 sous le titre "Molécules organiques et traitement du signal optique", le développement des télécommunications optiques requiert la réalisation de composants utilisant des matériaux de forte susceptibilité non linéaire du deuxième ou du troisième ordre.

De nombreux composés tant inorganiques qu'organiques sont utilisés sous différentes formes telles que solutions, polymères, polymères dopés, couches monomoléculaires, cristaux liquides, monocristaux, polymères cristaux liquides ou analogues.

Les composés organiques sont très intéressants car la synthèse d'une très grande variété de produits est généralement possible. De plus, la plupart des composés organiques présentent une résistance élevée aux agressions extérieures (humidité, acidité, oxydation, etc) et peuvent être ajoutés dans des matières telles que des films polymériques ou analogues, permettant une mise en forme aisée.

J.F. Nicoud et R.J. Twieg dans leur mémoire intitulé "Design and synthesis of organic molecular compounds for efficient second harmonic generation" Ed. D.S. CHEMLA et J. ZYSS, 1987 répertorient différentes molécules susceptibles d'être actives en optique non linéaire.

Ces molécules ont comme squelette des chaînes carbonées comprenant généralement des cycles aromatiques substitués d'une part par des groupements donneurs d'électrons et d'autre part par des groupements accepteurs d'électrons.

La délocalisation des électrons engendre de forte hyperpolarisabilité du troisième ainsi que du second ordre lorsque la molécule est non-centrosymétrique.

Des efforts de recherche importants sont continuellement développés pour découvrir et fabriquer de nouveaux composés présentant une activité en optique non linéaire.

Ainsi, l'invention propose de nouveaux composés thiophéniques qui présentent notamment une activité élevée en optique non linéaire.

A cet effet, l'invention propose de nouveaux composés thiophéniques de formula I suivante :

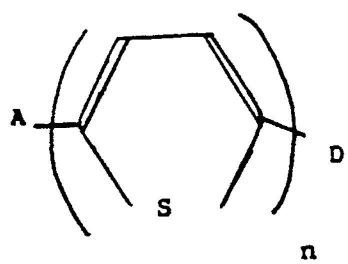

(I)

dans laquelle :

- n est un nombre entier compris entre 1 et 10 avantageusement de 1 à 4 ;
- D représente un groupement donneur d'électrons choisi parmi les groupements de formules suivantes :

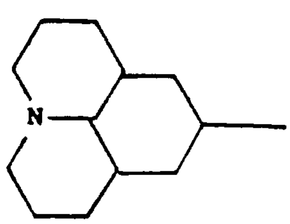

ou $-R_2(D_1)_m$

(II)

Dans laquelle $R_2$ est un radical aryle, de préférence le radical phénylène, m est un nombre entier égal à 1,2 ou 3 et $D_1$ est un radical choisi dans le groupe comprenant les radicaux amino, alkylamino,

dialkylamino arylamino, hydroxyle, thiol, alkylthio, arylthio, alkoxy, alcoxyphényle, aryloxy halogenoalkyle, oxy, et

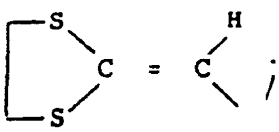 ; 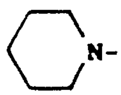 ; 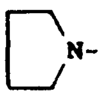 ; 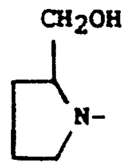 ; 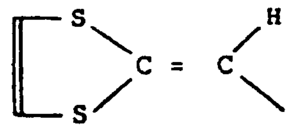

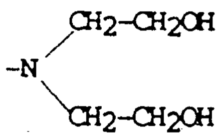

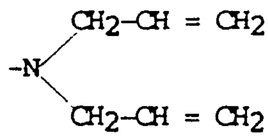 ; 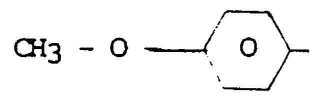

- A représente un groupement accepteur d'électrons choisi parmi les radicaux nitro, cyano ou de formule générale.

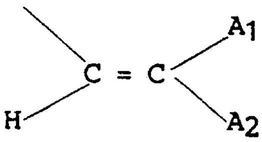

(III)

dans laquelle $A_1$ et $A_2$ identiques ou différents représentent l'hydrogène, un radical cyano, nitro, phényle - sulfone, paranitrophényle ou - $PO_3(R_5)_2$ dans laquelle $R_5$ est un radical alkyle inférieur tel que le radical éthyle ou propyle. Toutefois, les groupements $A_1$ et $A_2$ ne peuvent simultanément représenter l'hydrogène.

Selon une caractéristique préférée de l'invention, les radicaux D ont les formules suivantes :

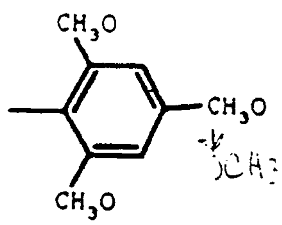

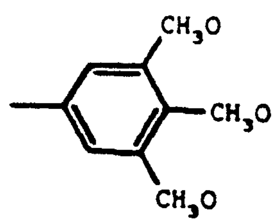

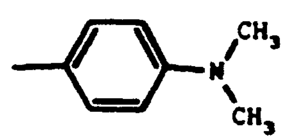

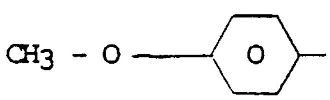

Selon une autre caractéristique de l'invention, les radicaux $A_1$, $A_2$ préférés de l'invention sont les radicaux cyano et nitro, et avantageusement les couples cyano/cyano et cyano/nitro.

Les composés de l'invention sont, par exemple, les composés de formules suivantes :

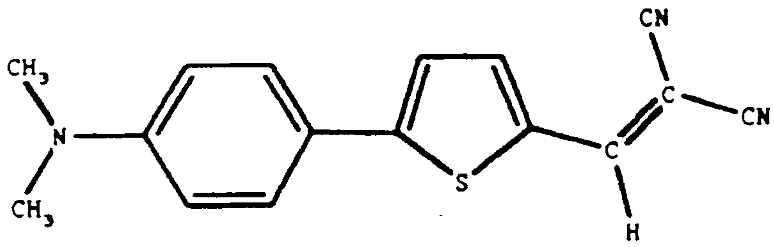

(i)

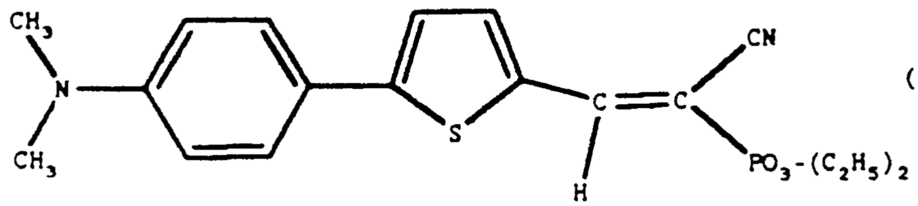

(ii)

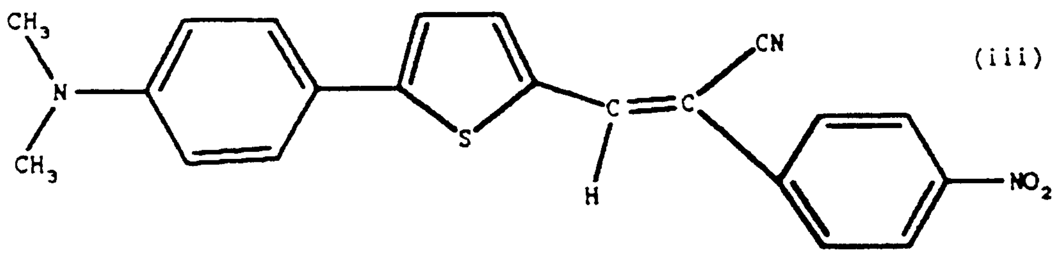

(iii)

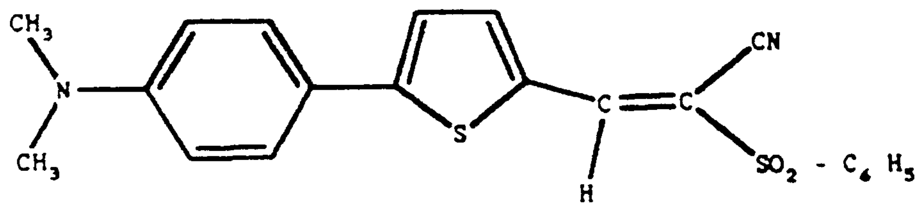

(iv)

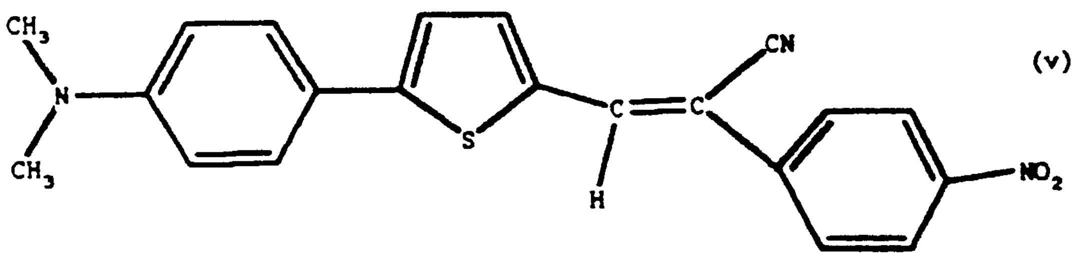

(v)

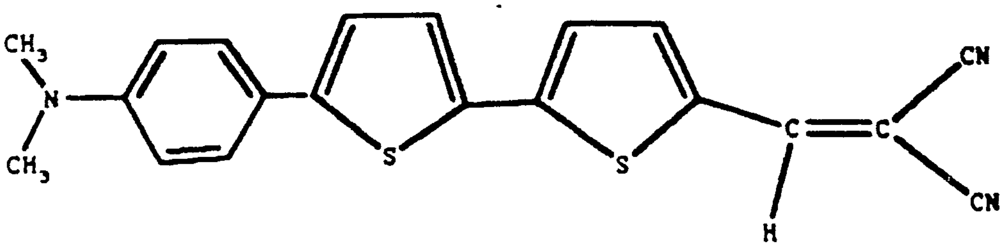

(vi)

Ces composés peuvent être obtenus selon différents procédés de synthèse.

L'invention a également pour objet des composés à hyperpolarisabilité élevée, actifs en optique non linéaire de formule suivante :

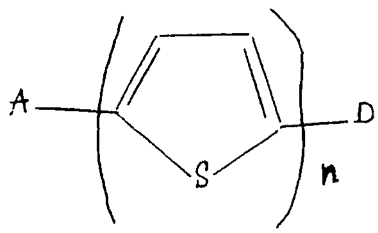

dans laquelle : n est un nombre entier compris entre 1 et 10

A est un groupement accepteur d'électrons

D est un groupement donneur d'électrons

Selon une autre caractéristique de l'invention, les groupements A et D ont les significations indiquées précédemment.

Avantageusement les composés de l'invention à forte hyperpolarisabilité sont les composés de forme trans.

Les composés de l'invention ont la propriété d'être actif en optique non linéaire et donc sont susceptibles d'être utilisés dans des dispositifs de traitement électrooptique ou purement optique, notamment dans le domaine des transducteurs, des modulateurs, amplificateurs etc...Dans l'application au doublement de la fréquence, on préfère que n soit compris entre 1 et 4 avantageusement de 1 à 2. En effet, le doublement d'harmonique exige de la transparence au fondamental comme en 2ième harmonique et le $\lambda$ max doit être sensiblement inférieur à 450 nm pour un laser de pompage à 1060 nm. En revanche, dans les applications telles que des dispositifs de traitement électrooptique et l'amplification parmétrique, le $\lambda$ max peut largement excéder cette valeur de 450 nm

En effet, l'activité des matériaux en optique non linéaire est déterminée par la valeur des coefficients et d'hyperpolarisabilité des second, troisième ou énième ordre.

L'hyperpolarisabilité d'un composé est directement reliée au moment dipolaire moléculaire par la relation fondamentale

$\underline{\mu} = \underline{\mu o} + \alpha . \underline{E} + \beta \, \underline{E.E} + \gamma \, \underline{E.E.E} + ...$

dans laquelle : $\underline{\mu}$ et $\underline{\mu o}$ représentent les moments dipolaires respectivement en présence et en absence de champ électromagnétique.

E représente le champ électrique ou électromagnétique local d'excitation.

$\alpha$ , $\beta$ , $\gamma$ représentent les coefficients de polarisabilité et d'hyperpolarisabilité.

En effet, le coefficient $\alpha$ est le coefficient de polarisabilité de la molécule et reflète son activité en optique linéaire.

Les coefficients $\beta$ et $\gamma$ représentent les coefficients d'hyperpolarisabilité respectivement de second et troisième ordre.

Ces coefficients reflètent l'anharmonicité du potentiel électrique dans la molécule et sont fortement dépendants de la symétrie et de la structure de celle-ci.

Par ailleurs, les coefficients d'ordre impair, tel que le coefficient $\gamma$ ne sont jamais nuls pour toutes molécules. Au contraire, les coefficients d'ordre pair tel que le coefficient $\beta$ sont nuls pour les molécules centrosymétriques.

Il est intéressant d'utiliser des molécules présentant un coefficient d'hyperpolarisabilité non nul pour les applications en optique non linéaire telles que, par exemple, les dispositifs électrooptiques, les modulateurs électrooptiques, les amplificateurs paramétriques, les dispositifs doubleur de fréquence ou analogues.

Pour apprécier et mesurer le coefficient $\beta$ des molécules, on détermine celui-ci par comparaison avec celui d'une molécule de référence, à savoir, l'urée.

La mesure de l'hyperpolarisabilité moléculaire $\beta$ d'un composé peut être réalisée généralement dans une expérience de génération de deuxième harmonique. Elle se déroule en milieu solvant tel que, par exemple, l'acétone, l'eau, le diméthylsulfoxyde. La méthode connue sous le nom de méthode EFISH, est décrite dans les articles B.S. LEVINE et al Appl. Phys. Lett. vol 24 p. 445, 1974, et J.L. HOUDAR et al. J. Chem. Phys. vol 67 p 1926, 1977.

Il est également possible de mesurer le produit $\mu \, \beta$ (- $\omega$ ;$\omega$,O) par la mesure de la susceptibilité

électrooptique $\chi^{(2)}$ (-ω ;ω . O) d'un film dopé et polarisé de PMMA contenant N molécules actives par unité de volume. La measure de $\chi^{(2)}$ (-ω ;ω . O) peut être réalisée par interférométrie comme cela est décrit dans l'article de K.D. SINGER et al J. Opt. Soc. Am. B vol 4 n° 6 p. 968 et s. (1987).

La relation entre μβ et $\chi^{(2)}$ est bien connue, on la trouvera par exemple dans l'article de K.D. SINGER et al. Appl. Phys. Lett. vol 49 n° 5 p. 248 et s. (1986).

L'hyperpolarisabilité de la molécule peut également être exprimée par un coefficient β μ statique qui correspond à l'activité de la molécule à la fréquence nulle et donc donne une mesure de l'activité intrinsèque de la molécule.

Pour cela, on réalise une mesure de β μ à une fréquence donnée, à l'aide d'un interféromètre MACH-ZENDER, puis on ramène cette valeur à une fréquence hypothétique nulle au moyen de modèles de calcul dits "modèle à deux niveaux".

La méthode de mesure et de calcul du β μ statique est décrite dans l'article déjà cité de K.D. SINGER et al paru dans J-OPT.SOC.AM B vol 4 n° 6 pp. 968 et s (1987).

En outre, il est intéressant que les composés actifs en optique non linéaire présentent une bonne photostabilité.

En effet, comme cela est décrit dans l'article déjà cité de K.D. Singer et al J.Opt. Soc Am. B vol 4 n° 6 pp. 968 et s (1987), l'hyperpolarisabilité β (-ω ;ω .o) est fonction de la fréquence ω de la lumière utilisée : β sera d'autant plus élevée que la fréquence ω ou la longueur λ du rayonnement sera proche de la fréquence d'absorption ω max ou longueur d'onde maximale d'absorption λ max de la molécule.

Cependant, à une telle longueur d'onde ou fréquence, la plupart des molécules fortement conjuguées s'avèrent être peu stables sous un rayonnement laser.

Au contraire, les composés de l'invention présentent une remarquable stabilité photochimique même sous un rayonnement de longueur d'onde proche de la longueur d'onde maximale d'absorption λ max de la molécule.

L'invention sera plus clairement illustrée au vu des exemples donnés ci-dessous uniquement à titre indicatif.

Un autre but de la présente invention est de fournir un procédé de fabrication des composés selon la présente invention. Ces composés sont en effet facilement obtenus de manière originale au moyen d'un procédé comportant les étapes suivantes :

a) formation d'un organo-zincique duthiophène de préférence par action successive du butyllithium et d'un halogénure de zinc ;

b) réaction de l'organo zincique sur un réactif D-X où X est un groupe tel que par exemple un halogénure, chlorure ou bromure ;

c) anionisation du produit obtenu sur la deuxième position réactive du thiophène, de préférence par action du butyl lithium ;

d) réaction du carbanion obtenu en c) avec du diméthylformamide (D.M.F.) ;

e) réaction de l'aldéhyde obtenu un groupement méthylène activé susceptible de réagir avec de l'aldéhyde pour former une double liaison.

Le groupement méthylène activé étant de formule $A_1$-$CH_2$-$A_2$.

L'étape a) et l'étape b) peut être réalisée en utilisant les techniques générales décrites dans "tetrahedron letters", volume 28, numéro 43, page 5213 à 5216 en 1987. Les étapes c) et d) sont la transposition de la formation d'un aldéhyde selon la technique décrite dans "Synthesis, 228 (1984)" dans J. Org.Chem. 40,231 (1975: dans Synthesis 625 (1980).

Les polythiophènes réagissent comme le thiophène.

Exemple 1 : préparation du composé de formule (i)

Le procédé de fabrication décrit dans cet exemple peut être mis en oeuvre pour la synthèse des composés de formule (ii) à (v) par simple modification des produits de départ et des conditions opératoires.

a) Préparation de :

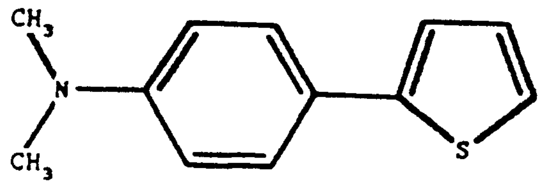

Dans un ballon, on introduit 0,7 mole de tiophène sec dans 250 ml de tétrahydrofuranne (THF) sec puis on introduit 0.64 mole de N.butyl lithium (1,6 M dans l'hexane) après avoir refroidi la solution à - 20 °C.

On dissout 0,7 mole de $ZnCl_2$ dans 500 ml de THF sec et on mélange les deux solutions. L'ensemble est maintenu sous agitation pendant environ 2 heures à température ambiante.

La solution obtenue est introduite dans le mélange : 0.00132 mole de Dibenzylcétone de palladium (Pd $(DBA)_2$), 0,00559 de triphénylphosphine (TPP) et 0,421 mole de parabromodiméthyl aniline. L'ensemble est porté à reflux 16 heures puis refroidi avant addition d'eau.

On procède à une extraction à l'acétate d'éthyle.

Le produit récupéré est séché sur Mg $SO_4$ puis recristallisé dans le méthanol. Le solide récupéré (44.64 g) a une température de fusion de 122 °C.

b) Préparation de :

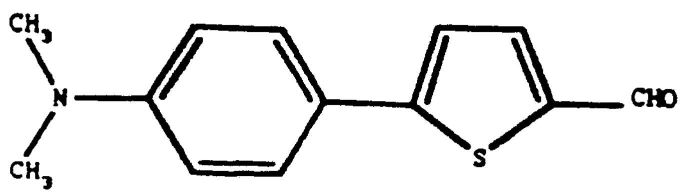

0,017 mole du produit obtenu en a) est dissous dans 200 ml de THF sec. A cette solution refroidie à 0 °C, on ajoute 10,6 $cm^3$ d'une solution de n-BuLi à 1,6 M dans l'hexane. Après 1 heure de réaction, on ajoute 2 ml de Diméthyl formamide et on laisse réagir 16 h à 25 °C.

Après acidification du milieu, on procède à une extraction par l'acétate d'éthyle. Après lavage à l'eau des phases organiques et séchage sur $MgSO_4$, on évapore le solvant pour récupérer un solide jaune de température de fusion égale à 196 °C.

Les analyses de spectrométrie infra-rouge, ultra-violette ou de RMN et spectrographie de masse confirment la structure indiquée ci-dessus.

c) préparation du composé de formule (i)

on introduit dans 120 ml d'éthanol, 9,28 x $10^{-3}$ mole de dycyanométhane, 4 gouttes de pipéridine et 5,54 x $10^{-3}$ mole du composé préparé en b).

Après un chauffage à reflux de 3 heures, on récupère le précipité rouge formé par filtration et lavage à l'hexane.

Le produit récupéré présente une température de fusion de 217 °C. Sa structure a été confirmée par analyses spectrographiques.

Il présente une longueur d'onde maximale d'absorption max en milieu $CHCl_3$ égale à 510 nm pour un coefficient d'extinction = 40 700.

<u>Exemples 2 à 5</u>

Les composés (ii) à (v) sont préparés de manière analogue au composé (i) , toutefois les composés mis en réaction avec le composé

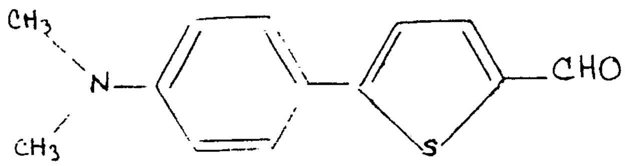

sont respectivement $CH_2$ -(CN) $PO_3$ $(OEt)_2$ :

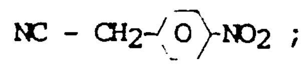

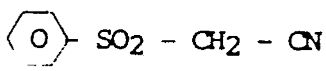 ; et 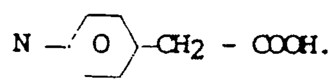

Les structures des produits obtenus ont été vérifiées par analyses spectrales et élémentaires.

Les caractéristiques des produits obtenus sont les suivantes :

ex 2 prod. (ii) pf : 156 °C λ max ($CHCl_3$) = 464 nm ε = 25900

ex 3 prod. (iii) pf : 298 °C λ max ($CHCl_3$) = 499 nm ε = 37500

ex 4 prod. (IV) pf : 211 °C λ max ($CHCl_3$) = 498 nm ε = 36400

ex 5 prod. (V) pf : 240 °C λ max ($CHCl_3$) = 457 nm ε = 28200

## Exemple 6

De manière analogue aux exemples précédents, on fait réagir le composé de formule (0,020 mole)

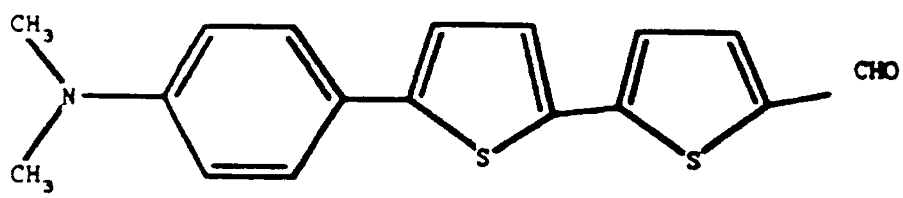

dissous dans 250 ml d'éthanol avec 0,022 mole de $NC-CH_2-CN$, en présence d'une quantité catalytique de pipéridine. Après chauffage à reflux pendant 5 heures, on récupère le précipité rouge-brun formé par filtration, lavage à l'hexane et séchage.

Sa structure a été vérifiée par analyses spectrales. Il présente un point de fusion égal à 215 °C et un max ($CHCl_3$) = 540 nm.

## Exemple 7 : Synthèse de composé de formule :

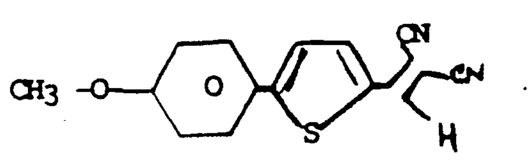

Composé VII

La synthèse comporte les 3 étapes réactionnelles suivantes :

a) 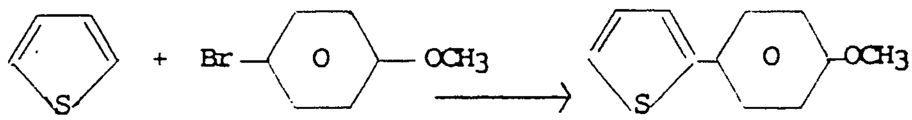

Composé intermédiaire 1

b) 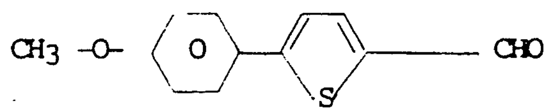 (2)

Composé intermédiaire 2

c)

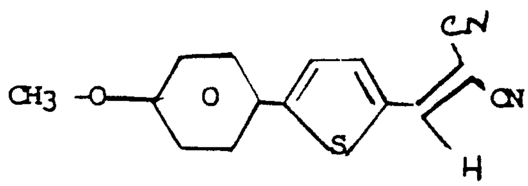

Composé VII

a) Synthèse du paraanisyl-2 thiophène

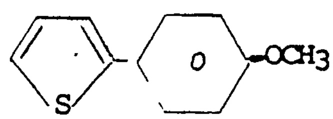 (1)

Dans un ballon tricol de 500 ml placé sous azote, on introduit 23,30 g de thiophène (0,277 mole) et 140 ml de THF sec ; on refroidit à -20 °C et on coule lentement 160 ml de Butyl-lithium (46M/l dans l'hexane) ; on laisse remonter à 0 °C ; durée 2 h 30.

Dans un tricol d'un litre sous agitation mécanique, on charge sous azote 34,6 g (0,254 M) de $ZnCl_2$ et 275 ml de THF sec. On laisse dissoudre et on coule en 30 mn la solution précédente ; la réaction est légèrement exothermique. On rajoute 20 ml de THF pour le rinçage.

Dans un tricol de 2 litres placé sous azote, on charge 0,3907g ($6,80 \times 10^{-4}$ mole) de Pd $(DBA)_2$, 0,714 g ($2,72 \times 10^{-3}$ mole) de TPP et 220 ml de THF sec ; on laisse réagir 10 mn et on rajoute 45,9 g, (0,245 mole) de parabromo anisole et on coule à la température ordinaire la solution précédente ; on porte à reflux 16 heures. On laisse refroidir, on rajoute de l'eau et on extrait à l'acétate d'éthyle ; on lave à l'eau ; on sèche sur $MgSO_4$ ; on évapore, on récupère un solide. Par recristallisation, on obtient 27,3 g d'un solide (Point de fusion : 111 °C) (paramethoxyphenyle- 2 thiophène)
Rendement isolé : 58,6 %

b) Synthèse de (2) :

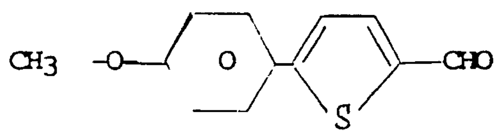

(2)

Dans un tricol de 500 ml sous azote, on introduit 20,4 g (0,1073 mole) paramethoxyphenyle- 2 thiophène obtenu à l'étape précédente et 170 ml de THF sec : on refroidit à -5,-10 ° C et on coule en 30 mn une solution de butyl-lithium hexane (1.6 M), 0.0972 mole de n butyl-lithium. On laisse 3 heures à 0 ° C.

On introduit ensuite 9,7 g (0.133 M) de Diméthyl formamide (DMF) sec. On laisse réagir à température ambiante 16 heures.

On hydrolyse, on extrait à l'acétate d'éthyle ; on sèche sur $MgSO_4$ et on récupère 368 g d'un solide orange.

On rajoute de l'hexane chaud et on récupère par filtration 16,8 g d'un solide rose pâle (point de fusion = 120-122,5 %)

Rendement isolé : 79,3 %

c) Synthèse de (VII)

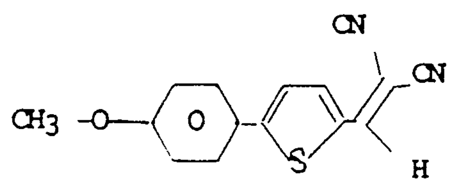

(7)

Dans un tricol de 1 litre sous azote, on introduit (0,9 g (0,05 mole) de (2) :

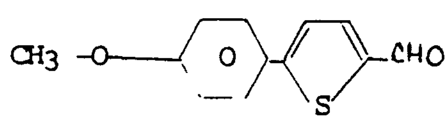

et 580 ml d'éthanol absolu. On chauffe à 50 ° C pour solubiliser (2) ; on rajoute ensuite 5,9 g (0,0893 mole) de $CN\text{-}CH_2\text{-}CN$ et 10 gouttes de pipéridine.

On chauffe 5 mn à reflux ; il y a formation du précipité orange ; on laisse à reflux 3 heures.

On filtre ; on laisse à l'hexane et on sèche 16 h au dessicateur. On récupère 11,9 g d'un solide orange.- (point de fusion : 195 ° C)

Rendement isolé (7) : 89,5 %

Les analyses en infra-rouge, ultra-violet, résonnance magnétique nucléaire du proton, confirment la structure des produits.

λ max ($CHCl_3$) : 430 nm

(ε : 36 900)

Les résultats de la détermination du coefficient d'hyperpolarisabilité et du coefficient statique $\beta\,\mu$ de certains de ces produits selon les méthodes décrites précédemment sont rassemblés dans le tableau ci-dessous.

| Ex. | Composé | Coefficient d'hyperpolarisabilité $\beta$ (w,w,o) à $\lambda$ = 633 nm | Coefficient statique $\beta$ $\mu$ x $10^{-48}$ e.s.u. | $\lambda$ max |
|---|---|---|---|---|
| 1 | (i) | 7630 x $10^{-48}$ e.s.u. | 1200 | 510 nm + |
| 3 | (iii) | 1980 x $10^{-48}$ e.s.u. | 510 | 490 nm |
| 6 | (vi) | 11500 x $10^{-48}$ e.s.u. | 1 125 | 540 nm + |
| 7 | (vii) | 2500 x $10^{-48}$ e.s.u. | 850 | 450 nm |

Ces composés présentent également une très bonne stabilité photochimique. Ainsi, sous un rayonnement laser de 633 nm de longueur d'onde et 5 mwatt.$mm^{-2}$ de puissance, un matériau comprenant du "Disper Red One" ou DR1 (nom commercial de la Société Aldrich) dispersé dans une matrice de PMMA d'épaisseur 14 $\mu$m présente une diminution du coefficient d'extinction après seulement 1 mn d'exposition, égale à 10 %.

Au contraire, avec les composés de l'invention, une telle diminution n'est pas observée même après 16 heures d'exposition.

Les composés de l'invention sont utilisés dans des composants de dispositifs électrooptiques ou optiques sous forme de matériaux, tel que par exemple sous forme de film, par incorporation dans une matrice, tel qu'un polymère, une résine etc. selon des techniques classiques et connues.

Ainsi, à titre d'exemple, les molécules préparées selon les exemples 1 à 6 sont incorporées dans un film de polymère transparent de 0,5 à 200 $\mu$m d'épaisseur, comme cela est décrit dans le brevet européen n° 218938. Comme polymères convenables, on peut citer, par exemple, le polyméthylméthacrylate (PMMA), le polystyrène atactique.

Les résultats de la détermination du coefficient d'hyperpolarisabilité et du coefficient statique $\beta$ $\mu$ de certains de ces produits selon les méthodes décrites précédemment sont rassemblés dans le tableau ci-dessous.

Le film de polymère est chauffé à une température supérieure à sa température de transition vitreuse (Tg), puis soumis à un champ électrique intense pour orienter les molécules actives conformes à l'invention.

Le film est ensuite refroidi à une température inférieure à la température de transition vitreuse Tg pour ainsi geler la position orientée des molécules actives.

Un film contenant des molécules actives orientées de l'invention présente un coefficient électrooptique r et un coefficient de génération de second harmonique comparables à ceux des cristaux inorganiques habituellement utilisés dans ces applications tels que par exemple, le diphtalate de potassium, le diphtalate d'ammonium, le dihydrogénophtalate de potassium.

Le film présente, en outre, des avantages spécifiques tels qu'une basse constante diélectrique et une activité électrooptique d'origine essentiellement électronique.

Ces matériaux actifs en optoélectronique, notamment sous forme de film, sont susceptibles d'être utilisés dans des modulateurs électrooptiques, des guides actifs tels que des coupleurs directionnels, polariseurs, modulateurs intégrés etc...

## Revendications

1. Composés thiophéniques caractérisés en ce qu'ils ont la formule générale suivante :

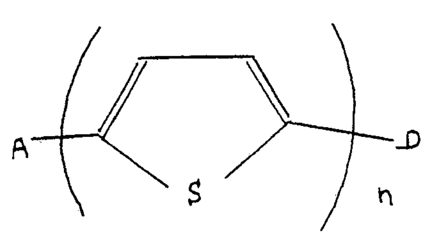

(I)

dans laquelle :

- n est un nombre entier compris entre 1 et 10 avantageusement compris entre 1 et 4.
- D représente un groupement donneur d'électrons choisi parmi les groupements de formules suivantes :

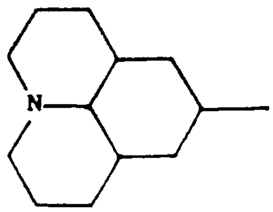

ou $-R_2(D_1)_n$

(II)

dans laquelle : $R_2$ est un radical aryle, de préférence le radical phénylène et $D_1$ est un radical choisi dans le groupe comprenant les radicaux amino, alkylamino, dialkylamino, arylamino, hydroxyl, thiol, alkylthio, arylthio, alkoxy, aryloxy halogénoalkyl, oxy, et notamment

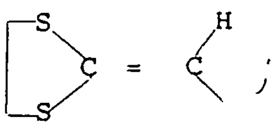 ; 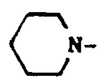 ; 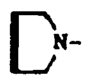 ; 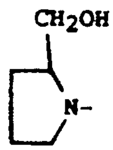 ; 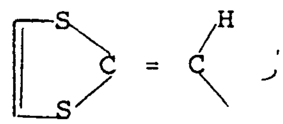 ;

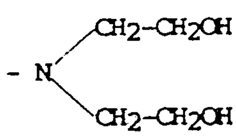 ; 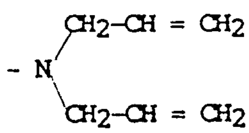 paraméthoxyphényl

- A représente un groupement accepteur d'électrons choisi parmi les radicaux nitro, cyano ou de formule générale

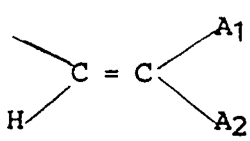

(III)

dans laquelle $A_1$ et $A_2$ identiques ou différents représentent l'hydrogène, un radical cyano, nitro, phényl sulfone, paranitrophényl ou - $PO_3(R_5)_2$ dans laquelle $R_5$ est un radical alkyle inférieur tel que le radical éthyle ou propyle. Toutefois, les groupements $A_1$ et $A_2$ ne peuvent simultanément représenter l'hydrogène.

2. Composés selon la revendication 1, caractérisés en ce que le groupement D est choisi dans le groupe comprenant les radicaux de formule

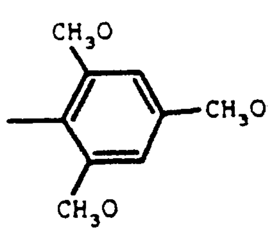 ; 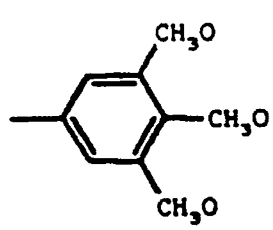 ; 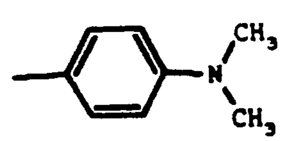

3. Composés selon la revendication 1 ou 2, caractérisés en ce que les groupements $A_1$ et $A_2$ sont choisis dans le groupe comprenant les radicaux cyano, nitro.

4. Composés selon la revendication 3, caractérisés en ce que $A_1$ et $A_2$ dans la formule III représentent respectivement les radicaux cyano/cyano, cyano/nitro ou nitro/cyano.

5. Composés selon l'une des revendications 1 à 4, caractérisés en ce qu'ils ont les formules suivantes :

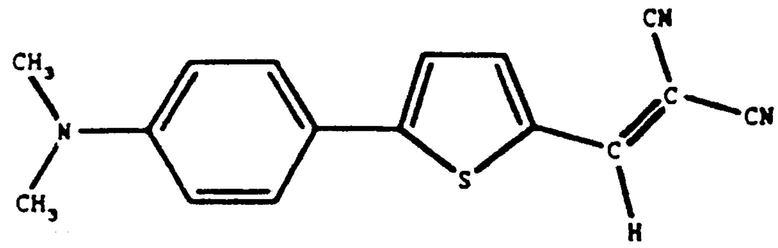

(i)

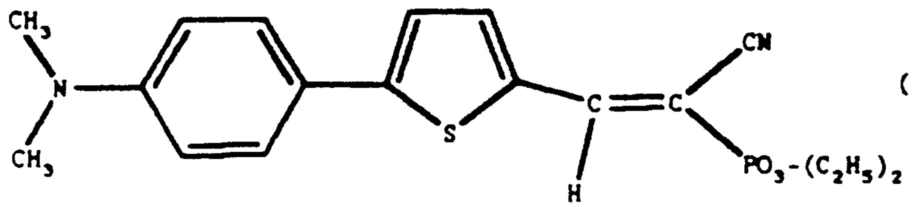

(ii)

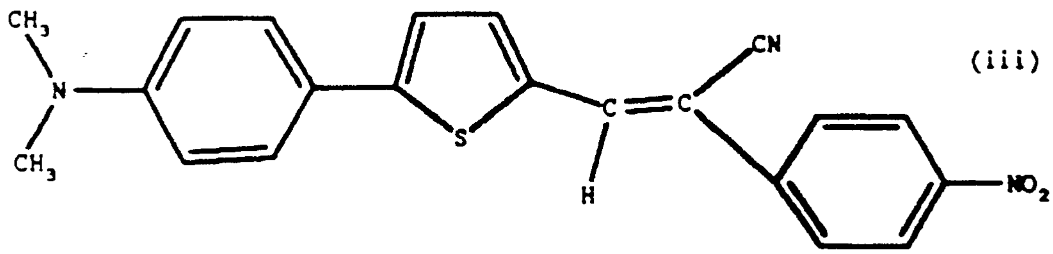

(iii)

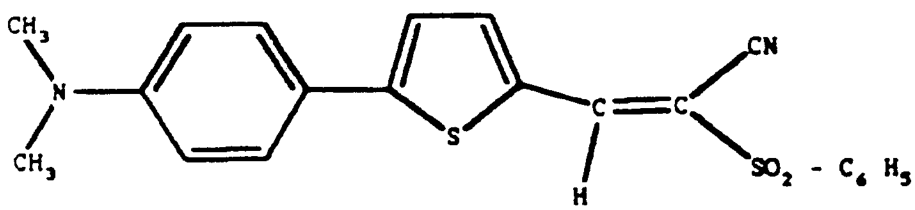

(iv)

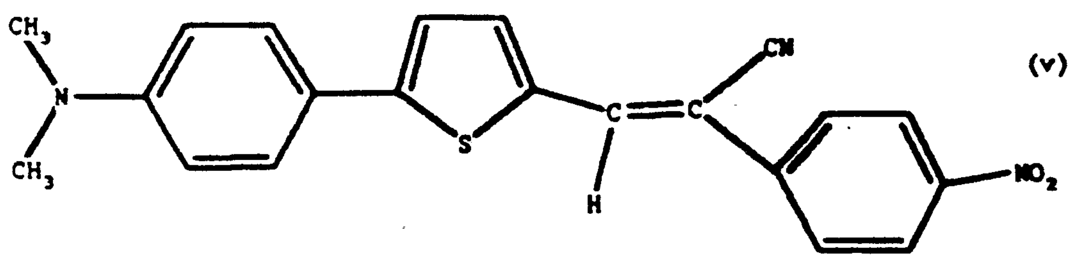

(v)

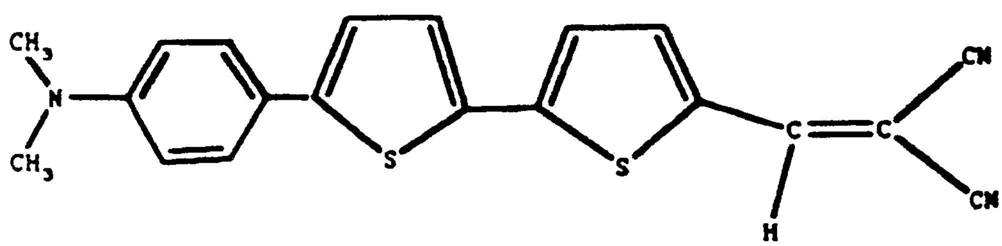

(vi)

6. Composés hyperpolarisables caractérisés en ce qu'ils sont des dérivés du thiophène de formule générale suivante :

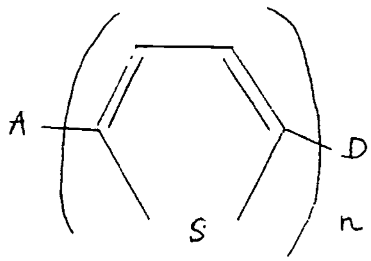

dans laquelle : n est un nombre entier compris entre 1 et 10 avantageusement compris entre 1 et 4.
A est un groupement accepteur d'électrons
D est un groupement donneur d'électrons

7. Composés selon la revendication 6 comprenant des radicaux A et D selon l'une des revendications 1 à 5

8. Matériau actif en optique non linéaire, notamment susceptible de générer une harmonique du second ordre, caractérisé en ce qu'il comprend au moins un composé selon l'une des revendications 6 et 7.

9. Matériau selon la revendication 8, caractérisé en ce qu'il est constitué par une matrice formée par un polymère, dans laquelle est incorporé au moins un composé selon l'une des revendications 6 et 7.

10. Matériau selon la revendication 8 ou 9, caractérisé en ce qu'il est sous forme de film.

11. Dispositif optique ou optoélectrique comprenant un composant actif en optique non linéaire caractérisé en ce que ce composant comprend un matériau selon l'une des revendications 8 à 10.

12. Procédé pour réaliser les composés selon les revendications 1 à 7, caractérisé par le fait qu'il comporte les étapes suivantes :

a) formation d'un organo-zincique duthiophène de préférence par action successive du butyllithium et d'un halogénure de zinc :

b) réaction de l'organo zincique sur un réactif A-X où X est un groupe tel que par exemple un halogénure, chlorure ou bromure ;

c) anionisation du produit obtenu sur la deuxième position réactive du thiophène, de préférence par action du butyl lithium ;

d) réaction du carbanion obtenu en c) avec du diméthylformamide (D.M.F.) ;

e) réaction de l'aldéhyde obtenu un groupement méthylène activé susceptible de réagir avec l'aldéhyde pour former une double liaison.

Le groupement méthylène activé étant de formule $A_1$-$CH_2$-$A_2$.

Office européen des brevets

# RAPPORT DE RECHERCHE EUROPEENNE

Numero de la demande

EP 90 40 0428

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 268 354 (I.C.I.)<br>* Revendications *<br>--- | 1,6,8 | C 07 D 333/24<br>C 07 F 9/40<br>G 02 F 1/35 |
| A | CHEMICAL ABSTRACTS, vol. 82, no. 11, 17 mars 1975, page 413, résumé no. 72683r, Columbus, Ohio, US; A. KRUTOSIKOVA et al.: "Furan derivatives. XLI. Synthesis and absorption spectra of 2-cyano-3[5-(substituted-phenyl)-2-furyl]acrylonitriles", & ZB. PR. CHEMICKOTECHNOL. FAK. SVST. 1972 (Pub. 1974), 49-52<br>* Résumé *<br>----- | 1 | |
| | | | **DOMAINES TECHNIQUES RECHERCHES (Int. Cl.5)** |
| | | | C 07 D 333/00<br>C 07 F 9/00<br>G 02 F 1/00 |

Le présent rapport a été établi pour toutes les revendications

| Lieu de la recherche | Date d'achèvement de la recherche | Examinateur |
|---|---|---|
| LA HAYE | 15-05-1990 | CHOULY J. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................................................
& : membre de la même famille, document correspondant